# EUROPEAN PATENT APPLICATION

(11) **EP 4 014 860 A1**
(43) Date of publication of application: **22.06.2022**
(21) Application number: 20214791.4
(22) Date of filing: 17.12.2020
(51) Int. Cl.: A61B 5/08, A61B 5/113, A61B 5/00, A41D 13/12

(54) **SYSTEM FOR MONITORING THE THORACIC EXCURSION TO DETECT RESTRICTION OF RESPIRATORY FUNCTION**

(71) Applicant: Respit S.r.l., 39022 Algund (IT)
(72) Inventor: PUTZER, Stephan, 39022 Algund (IT); TARFUSSER, Ivo, 39022 Algund (IT)
(74) Representative: Schwarz & Partner Patentanwälte GmbH

(57) **Abstract**

System (1) for monitoring the thoracic excursion of a subject, the system (1) comprising a monitoring device (2), comprising attaching means (4), integratable into a garment, a stretch sensor (5), and transmitting means (6), and a receiver unit (7), which is spatially divided from the monitoring device (2), wherein the stretch sensor (5) is for attachment to the subject's body to detect a signal corresponding to expansion and contraction of the thorax, and wherein the transmitting means (6) include evaluation means (13) to process the signal obtained from the stretch sensor (5), wherein the evaluation means (13) is configured to divide the expansion and contraction of the thorax into thorax excursions and interference signals, wherein the evaluation means (13) is configured to detect a triggering event, wherein the triggering event is reached as soon as the frequency of the thoracic excursion falls out of a predefined frequency range over a predefined time span, wherein the transmitting means (6) include an emitter (14) to emit a signal to the receiver unit (7) if the triggering event is detected, an wherein the receiver unit (7) is configured to receive said signal of the emitter (14) and to issue an alert signal.

## Description

The present invention relates to a system and method for monitoring the thoracic excursion of a subject, the system comprising a monitoring device, comprising attaching means, integratable into a garment, and a receiver unit, wherein the attaching means comprises a stretch sensor and transmitting means.

### BACKGROUND OF THE INVENTION

Breathing is, amongst others, a human vital sign. Several attempts have been made to address respiratory monitoring in diverse contexts possessing a variety of designs, features and having included diverse mechanisms or the like. As such, breathing activity is being controlled, e.g. due to medical indication, during sporting- or leisure activity which, among others, can take place outside as well as in water, such as swimming. The latter, should be approached with particular caution, especially when children exert activities in water. According to the World Health Organization (WHO) global appraisals are supposed to significantly underestimate current health issues related to drowning.

Unintentional drowning is a major reason of death worldwide, responsible for approximately 372,000 deaths globally per year. With 7% of all injury-related deaths it is a prominent killer ranking third among leading causes of unintentional injury deaths worldwide. Especially children, aged 4-14 and younger, as well as men are most susceptible of drowning. Thus, unintentional drowning can be considered as the most important non-illness related cause of death in children aged under 4 years, thereby vaulting this matter to a remarkable public health threat worldwide (Data obtained from the World Health Organization Inc., February 2020).

In essence, several regimes present a system for monitoring respiratory function with varying quality and/or benefit in terms of the scope of the present invention, such as DE 10 2008 042 554 A1 that discloses a monitoring device comprising a strain sensor with at least one elastically deformable electrode, an evaluation unit and an insulation layer comprising dielectric elastomer material in which the electrode is arranged. The monitoring device can be used to monitor at least one physiological parameter, in particular respiration. The strain sensor, a capacitive elastomer sensor, can be very easily integrated (for example by gluing) into stretchable textile fabrics such as a chest band. The evaluation unit can detect a change in length of the strain sensor based on a resistance and/or conductivity measurement.

U.S. Patent Application US 2019/0201772 A1 describes a sports-bra with an integrated elastic band that surrounds the upper body. It contains a respiration monitoring system consisting of two modules that are coupled wirelessly: I) a strain sensor that reacts to the extension or contraction of the upper body of the wearer, II) an electronic module comprising a processor and a haptic feedback device. The latter generates haptic feedback in the form of vibration, reminding the wearer to breathe. The respiratory sensor module's data cannot be sent or received to any additional external device, so this device is only used locally. The aim of this invention is to monitor breathing activity during sports activities (including yoga) and also to provide immediate feedback to the user if breathing has an irregular pattern.

Another disclosure, DE 42 29 073 A1, shows a measuring device for detecting and monitoring respiratory activity by respiratory movement in humans and animals, comprising sensors that detect and measure changes in chest or abdominal girth. The sensors are elastic conductor materials such as electrically conductive rubber, which can be covered with an elastic insulation and with which a change in resistance is detected. The sensors can be integrated into an elastic body band, which includes a buckle (similar to a watch strap) to adjust the length. The body band also includes a device housing (also similar to the housing of a wristwatch), which contains a button cell, electronics, an LCD display, radio transmission and the like.

DE 196 28 356 C2 reveals a device for detecting dangerous breathing patterns such as hyperventilation in sport and professional divers comprising two elastic straps with strain gauges placed on the diver's chest. When a dangerous breathing pattern is detected, this information is transmitted to the diver by means of a display or acoustic warning elements, for example.

EP 0 699 052 B1 describes a respiration monitoring device based on a pneumatic principle. It consists of a breathing pressure sensor connected to a pressure sensor device of the monitoring device (breathing monitor), which visualizes respiration curves. This enables the monitoring and differentiation of respiratory events. The respiratory pressure sensor, preferably an electret pressure transducer, encloses the user's upper body and consists of a flexible, elastic and hollow tube with a fluid channel. Because the tubing is flexible, the total volume of the fluid channel increases as the user inhales. A low-pass filter is coupled to the sensor circuitry to receive the signal from the sensor and remove signal artifacts configured to a cut-off frequency of 2.0-2.5 Hertz (Hz).

However, most of the above-mentioned devices are not applicable for preventing unintentional drowning due to several reasons. As such, several concepts address an approach that collects physiological parameters for evaluation and analysis in the context of sports, for instance to improve their performance. In particular, they cover different techniques for measurement, i.e. the pressure sensors in EP 0 699 052 B1, which encompass a merely very short cut-off of e.g. 2.0-2.5 Hz. And, if they possess readout systems, they provide on the one hand systems to normalize breathing pattern for e.g. professionals as in DE 196 28 356 C2 and on the other hand systems stimulating and/or influencing the user, such as haptic feedback mechanism or transmission of irregular breathing activates locally and directly to the wearer himself (US 2019/0201772 A1) which are, in some embodiments, conceived from a single unit. Moreover, the devices disclosed in DE 10 2008 042 554 A1 and DE 42 29 073 A1 neither provide long-term usage in water nor encompass neutralization of interference signals tailored to the user. Apart from that and most importantly, prior art provides monitoring breathing activities, but hitherto does not solve the problem of a protective and thus life-saving regime, implying an medical utilizable alert signal and as a result thereof, do not intent to protect the user's life.

Hence, for these very reasons, taking account of the protective significance of respiratory monitoring devices as well as the scope, the challenge of introducing a life-saving system to detect a respiratory dysfunction in real-time in order to prevent unintentional fatal drowning, in particular well suitable for users such as children, remains high. Those above described advances provide on the one hand models to monitor vital signs such as the breathing activity but, on the other hand hurdles remain that, indeed, have to be solved. Consequently, there is a need in the art for improved benefit to subjects participating in water bathing activity to be continuously monitored by a system that emits a signal only in an emergency.

### SHORT DESCRIPTION OF THE INVENTION

The object of the present invention is to provide a monitoring regime to detect respiratory dysfunction during swimming- or dabbling activities in water for the prevention of unintentional fatal drowning, with particular emphasis on children.

The above defined problem is solved with the object of the present invention defined in the independent claims. Any further advantageous embodiments of the present invention are indicated in the dependent claims. The present invention provides a system for monitoring the thoracic excursion of a subject, the system comprising a monitoring device, comprising
- attaching means, integratable into a garment,
- a stretch sensor,
- transmitting means,
and a receiver unit, which is spatially divided from the monitoring device,
wherein the stretch sensor is for attachment to the subject's body to detect a signal corresponding to expansion and contraction of the thorax, and
wherein the transmitting means include evaluation means to process the signal obtained from the stretch sensor,
wherein the evaluation means is configured to divide the expansion and contraction of the thorax into thorax excursions and interference signals,
wherein the evaluation means is configured to detect a triggering event, wherein the triggering event is reached as soon as the frequency of the thoracic excursion falls out of a predefined frequency range over a predefined time span,
wherein the transmitting means include an emitter to emit a signal to the receiver unit if the triggering event is detected, and
wherein the receiver unit is configured to receive said signal of the emitter and to issue an alert signal.

Hence, the present invention provides a novel regime for monitoring one defined vital sign during swimming- or dabbling activities in water, which indeed advances a life-saving system by observing respiratory function to prevent unintentional fatal drowning of children.

Moreover, the object of the present invention provides preventing strategies for unintentional fatal drowning by a system for monitoring for use in water. As such, respiratory frequency is captured, processed and transmitted and most importantly, at any time and if necessary, an alert signal is emitted in real-time to the spatially divided receiver unit.

The stretch sensor, attached to the subject's torso, possesses the capacity to register every excursion of the subject's thorax. The processed signal, which is initially captured by the stretch sensor, draws conclusion about the subject's vital status.

The evaluation means is configured to distinguish between breathing activity and motor activity, resulting in a reliable system for monitoring. As a further advantage of the present invention, the system has the potential to detect pathological breathing patterns, such as apnea or inverse breathing. The latter phenomenon is understood as a respiratory dysfunction reversing normal breathing pattern.

Especially preferred is an embodiment where the receiver unit is spatially divided from attaching means worn on the subject's torso and functions as a standalone. Measurement data generated during the monitoring are preferably not for long-term storage. This protects the subject, respectively the user and prevents the use of third parties. Thus, data integrity is guaranteed at any time.

In case an alert signal is outputted, this is done via a wireless connection, indicating that there is no need in locally connecting the receiver unit to attaching means. This is advantageous over prior art by providing any flexibility and in this regard being easily relocated from one pool to another and it is as well suitable for use in lakes or oceans.

In another embodiment, the transmitting means is removably attached from the stretch sensor. In this regard providing flexibility in terms of laundry or charging reasons. Preferably, transmitting means may also include a button, which may be an electronically or electromechanical driven, to switch on/off the system for monitoring. For instance, it may be configured to press the button for a predefined time range to switch it on/off This is for safety reasons to prevent any unintentional deactivation of the monitoring system due to unfavorable movements by the subject. Further, transmitting means may also encompass a feedback mechanism to the receiver unit. This is to perform safety checks in order to assure an intact communication between the two of them. For example, the transmitting means may send a "SYSTEM OK" signal to the receiver unit, e.g. system ID and/or geographical location, at regular intervals, e.g. essentially every 60 seconds. This assures to communicate proper function of the emitter and, hence, to prevent false negative events (i.e. undetected emergency). When the receiver unit obtains the next "SYSTEM OK" signal, the previous one will be superseded. If the interval is exceeded without receiving any further signal, dysfunction of the transmitting means is assumed and an immediate check is required. Preferably, the previously transmitted location of the subject wearing the system is used, to track the subject as quickly as possible.

In a preferred embodiment, said triggering event is a respiratory frequency dysfunction, which is understood as an imminent restriction of respiratory function meaning that the subject is not breathing at a regular pattern. When a subject is supposed to drown, the respiratory frequency changes dramatically. The breathing pattern during dyspnea is commonly inspiratory followed by closing the trachea in the expiratory phase. Terminal breathing takes place roughly one to two minutes before the circulation finally ends and the subjects drowns. The situation is further aggravated by the fact that those, who do not get enough air to breathe cannot call for help. Thus, the present invention strives to detect near-drowning in order to be able to intervene as early as possible and ideally prevent a cardiovascular arrest. However, in the event of an arrest, the present invention may provide a massively shortened timeframe until resuscitation measures are initiated. Consequently, chances of survival can be improved, preferably without any neurological deficiencies.

In a preferred embodiment, said evaluation means is configured to transmit a signal to the receiver unit if a value of respiratory frequency as an indicator of potential respiratory dysfunction has been detected. Advantageously, the evaluation means is not continuously emitting any normal breathing pattern to a receiver unit. This system is therefore user friendly without any complexities and additionally prevents from being littered with unnecessary information. Furthermore, this principle is much more energy efficient. This is an additional advantage, as the monitoring device may have limited battery capacity.

In another preferred embodiment, said evaluation means is configured to learn about the subject's normally distributed respiratory frequency over a predefined time span. Preferably, it is also configured to differentiate between relatively constant rate of normal respiratory frequency and respiratory frequency dysfunction to detect alterations in breathing patterns.

Further, in a preferred embodiment, following variables may be amended for the capture of the signals via the stretch sensor and the evaluation of the captured signals via the evaluations means. For capturing the basic signal capture frequency may be amended, preferably it ranges from 5-50 Hz, more preferably from 10-50 Hz. The signal may be captured at several capture frequencies simultaneously. For evaluation of the captured signals the timeframe of primary smoothing of sequential signals, the number of sequential averaged values to compute the direction thereby indicating an ongoing phase of respiration as well as the percentage of previous amplitude (relative distance from the turning point) that will trigger an inspiration or expiration event can be amended. Finally, also age related limit values for respiratory frequency dysfunction can be taken into account. These variables are chosen such that the evaluation means allows neutralizing interference signals that do not correspond to thorax excursions effectively.

In a preferred embodiment, said transmitting means is configured to transmit the signal to the receiver unit wirelessly and in real-time. The real-time transmission assures the supervisor that, in any case, if the subject wearing the monitoring system, an immediate reply is carried out such that the time can be greatly reduced in order to administer first aid earliest possible for the drowning subject. The system is a valuable asset that helps to get the alert signal much earlier than by randomly scanning the pool/lake/ocean environment.. And, as already discussed above, time is precious in terms of unintentional drowning and subsequent resuscitation attempts.

In a preferred embodiment, said alert signal is a perceptible pattern, such as audibly or visually, allowing the user complete flexibility and the possibility to personalize how the alert signal should be issued, adapted to any situation. In another preferred embodiment, said receiver unit comprises a visual readout such as a screen, a display, a pilot- or warning lamp. Advantageously, it is not required to carry the receiver unit constantly on one's person or rather than permanently observing the receiver unit in order to prevent missing out on a potential alert signal. As an example, a warning lamp may be installed on the poolside coupled with an additional signal emitted to a screen, hence, thereby assuring that if an alert signal is emitted, it can't be overlooked.

In a preferred embodiment, said stretch sensor comprises an elastomer stretch sensor with at least one electrode preferably three thereof. The underlying mechanism of this stretch sensor is based on the resistance change caused by stretching the conductors of the stretch sensor.

Preferably, the stretch sensor changes its capacitance during deformation. Hence, by measuring the capacitance of the stretch sensor, the amount of deformation can be directly deduced.

These dielectric sensors provide high quality as well as particularly accurate measurement data by gathering the changes of the thorax during breathing. Due to their small size, they represent the ideal component to be integrated into an attaching means or a garment in a space-saving way. They further offer an extremely high wearing comfort and at the same time extremely valid measurements, which can be optimally connected to a transmitting means.

In a preferred embodiment, said garment further comprises a stretchable item of clothing to embrace the torso of the subject's body, such as a belt integratable into a top. In some extent, the attaching means is embracing the subject's torso. The garment may conceive a base layer of clothing such as a bikini top for girls/women and a T-shirt for boys/men suitable for water activities. Moreover, the belt is preferably tightly attached, though not pinching the skin, to the subject's body as well as invisibly encapsulated at the correct anatomical position of the subject's torso (thoracolumbar) to avoid any shifting of the stretch sensor. However, the belt must move with the subject's torso in order to allow sensing of the excursion as well as contraction of the subject's thorax followed by capturing accurate and precise data. In the utmost embodiment, the girth should not be perceived as disturbing by the subject.

In a preferred embodiment, said stretch sensor and transmitting means are water-repellent, water-resistant, waterproof or a combination thereof. Since the present invention aims to a usage in water for over a longer period of time, it is especially preferred that the mentioned components are resistant to water to guarantee a smoothly procedure of monitoring.

In a preferred embodiment, said system further comprises tracking means configured to physically locate current geographical location, especially when the monitoring device is worn by the subject. What is needed, is a system for not only monitoring the breathing activity during water activities, likewise also a simultaneous tracking module, such as GPS or other suitable location system, e.g. Bluetooth Low Energy (BLE). Preferably, the tracking means is configured to exclusively send a location signal if an alert signal, due to respiratory dysfunction, is emitted. This preferred embodiment drastically decreases the timeframe of localizing the drowning subject, in worst cases already drowned on the bottom of the pool. Thereby, as described above, saving precious minutes in surviving without sequelae such as neurological disabilities.

Moreover, the problem according to the invention is also solved by a method for monitoring the thoracic excursion of a subject, using a system according to the invention, comprising the steps:
- perceiving and transmitting expansion and contraction of the subject's thorax;
- dividing the expansion and contraction of the thorax into thorax excursions and interference signals;
- calculating the subject's thoracic excursion;
- determining if the triggering event is induced; and
- transmitting a signal in real-time to the receiver unit which is instantly outputting an alert signal if the triggering event is occurring.

The method includes sensing for thoracic expansion and contraction thereby indicating a starting point of monitoring the subject's respiratory frequency and assuring that the predetermined criteria are appropriate. In sensing of events and in response to determining that the pre-defined criteria of uncritical breathing frequency takes place, the method further covers a prompt real-time alert signal to the receiver unit. In a preferred embodiment, the predetermined criteria constitute of a determination of a certain limit value that the subject has not taken a breath within a pre-defined time. The real-time alert signal emitted by the receiver unit increases the chances of survival of the subject and thus considerably decreases the timeframe of being without oxygen and consequently preventing severe brain damage.

Thereby, the stretch sensor of the inventive system perceives the expansion and contraction of the subject's thorax and sends the measured signal to the transmitting means. Preferably, the capacitance of the stretch sensor is measured, which relates directly to the expansion and contraction of the subject's thorax, which trigger the deformation of the stretch sensor. At the transmitting means, the signal is processed with the help of the evaluation means. The evaluation means, on the one hand, divides the measured signal corresponding to the expansion and contraction of the thorax into thorax excursions and interference signals. Thus, it can calculate the subject's thoracic excursion. On the other hand, the evaluation means determines if the triggering event is induced.

In a particular preferred embodiment, said evaluation means is subtracting interference signals out of the group comprising muscle activity when moving arms and upper body, laughing, coughing, sneezing, screaming, crying or agitation from the expansion and contraction of the thorax. Preferably, the evaluations means is smoothing the measured values corresponding to the basic signal, which can be the capacitances of the stretch sensor. The smoothing may be done by averaging a certain number of measured values, depending on the capture frequency of the measured values. After smoothing the measured values the amplitudes of the breathing cycles can be obtained. These amplitudes change with each breathing cycle, but are significantly larger than the intercurrent deflections from coughs, sneezing, movements etc. There might be interference signals having a larger deflection, which cannot be filtered out in their entirety. However, they play a marginal role in determining the breathing frequency, as only prolonged breathing breaks shall induce the triggering event. Noteworthy, other methods for filtering interferences signals, such as value changes over time or inversion of delta over time, have also been probed. Even if these approaches work as well, they do not constitute a preferred embodiment. The subtraction method is advantageously, since it is not susceptible to artifacts and provides reliable results. Moreover, it prevents false negative results, which would lead to unrecognized breathing breaks.

In essence, these paradox thorax excursions are neutralized in order to prevent measurement errors and also false positive alert signals. This is of great importance in the case of a swimming subject. The swimming movement causes many interference signals by moving the arms and the upper body. The evaluation means allows to filter the expansion and contraction of the subject's thorax corresponding to the swimming movement and other possible interference signals out of the signal measured by the stretch sensor. In a further embodiment, the evaluation means can distinguish between the subject being at the swim or dipping briefly under water on purpose. In the latter case, no false positive alert signal is emitted.

According to another aspect of the present invention, said system is used for preventing unintentional fatal drowning as well as a variety of other settings, including utilization for personal security purposes for e.g. non-swimmers or personal emergency response. In an extended sense and due to the fact that according to latest numbers about 50% of drowning victims require hospitalization the monitoring system is configured at aiming i) for the prevention of unintentional fatal drowning, ii) saving lives, iii) simultaneously relieve clinical capacities like emergency department as well as care units.

Further, the object of the present invention presents a monitoring device configured to sense for expansion and contraction of the thorax by means of a starting point in order to monitor the subject's respiratory frequency indicated and assured by properly defined predetermined criteria. As configured, the monitoring device covers a prompt real-time alert signal to the receiver unit that senses events and responds to conclude that pre-defined criteria of uncritical breathing frequencies are met. In a preferred embodiment, the monitoring device is configured to employ that the predetermined criteria constitute a determination of a certain limit value that the subject has not taken a breath within a pre-defined time. In addition, the configuration of the monitoring device has the particularly advantageous effect that the real-time alert signal emitted by the receiver unit increases the chances of survival significantly and the timeframe without oxygen is remarkably reduced, thus avoiding severe brain damage.

In doing so, the stretch sensor of the monitoring device is configured to deliver the recognized subject's thoracic excursion to the transmitting means, which is configured to processes the signal with the aid of the evaluation means. Advantageously, the evaluation means is configured to classify the measured signal corresponding to the expansion and contraction of the thorax into thoracic excursion and interference signals. Based on that differentiation, the monitoring system is configured to determine the subject's thoracic excursion and beyond that, has the potential to arrange whether a triggering event is elicited or not.

In another preferred embodiment, the monitoring device is configured to ascertain signal to noise ratio in the sense of differentiation from the effective thoracic excursion and interference signals. The latter implies muscle activity when moving arms and upper body, laughing, coughing, sneezing, screaming, crying or agitation from the expansion and contraction of the thorax.

As further described and of utmost importance, the monitoring device is configured to subtract interference signals to prevent measurement errors and thus false positive alert signals. In this regard, measurement errors are defined as paradox thorax excursions that imply muscle activity of the swimming subject when moving arms and upper body, laughing, coughing, sneezing, screaming, crying or agitation from the expansion and contraction of the thorax Preferably, the evaluation means is configured to filter the expansion and contraction of the subject's thorax corresponding to the swimming movement and other possible interference signals out of the signal measured by the stretch sensor. In a further embodiment, the evaluation means is configured to distinguish between the subject being at the swim or dipping briefly under water on purpose. Preferably, in the latter case, no false positive alert signal is emitted.

### DETAILED DESCRIPTION OF THE INVENTION

Further details and advantages of the present invention result from the description and the accompanying figures, which are incorporated herein. Together with the corresponding figure description, the illustrated drawings further serve to explain the principles of and to enable a person skilled in the art to use the present invention.

Thereby shows:
- Fig. 1a: a schematic illustration in front view of a subject using the system.
- Fig. 1b: a schematic illustration in back view of a subject using the system including the spatially divided receiver unit according to embodiments of the present invention.
- Fig. 2: a partial enlarged/detailed view of at least minimum requirements of the monitoring device according to embodiments of the present invention.
- Fig. 3: a partial enlarged/detailed view of one extended version of the monitoring device according to embodiments of the present invention.
- Fig. 4a: a diagram of a detected, digitized signal during motor activity before smoothing and neutralizing of interference signals.
- Fig. 4b: a diagram of the processed signal during motor activity after smoothing as well as neutralization of interference signals.
- Fig. 5a: a diagram of a detected, digitized signal during apnea before smoothing and neutralization of interference signals.
- Fig. 5b: a diagram of the processed signal during apnea after smoothing and neutralization of interference signals.

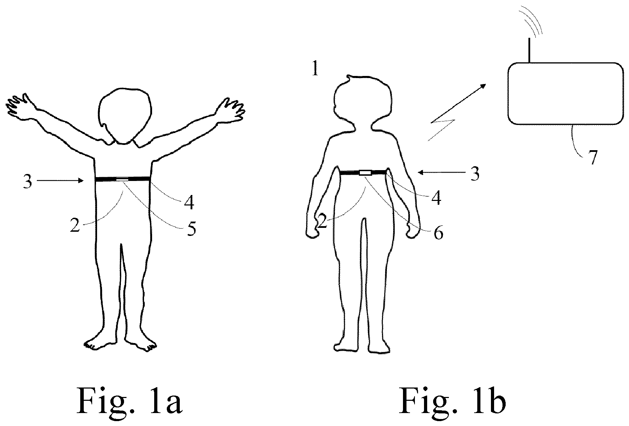

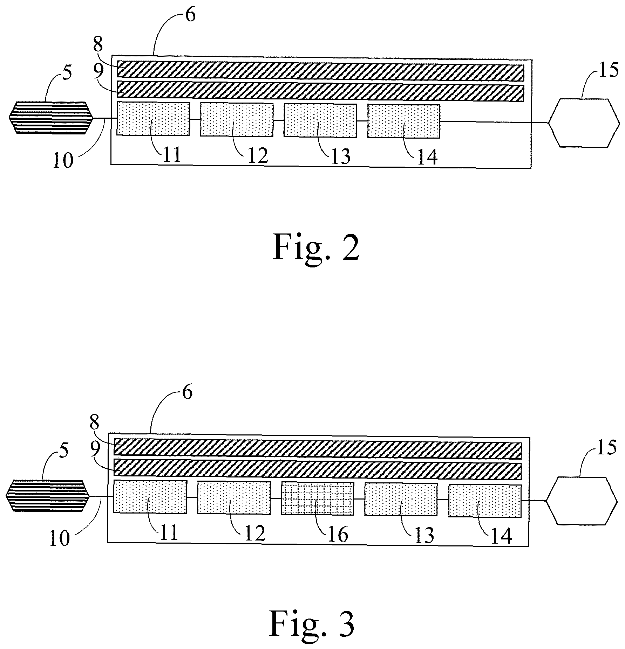

Fig. 1a and Fig. 1b illustrate a subject using the system **1** for monitoring that implies a monitoring device **3** consisting of attaching means **4** with stretch sensor **5** as well as transmitting means **6** and further including a spatially divided receiver unit **7.** The use of the system **1** for monitoring shall prevent the subject of unintentional fatal drowning. The attaching means **4** is worn on the subject's torso **2.** In some embodiments the attaching means **4** may be a belt integratable into a garment, particularly a top. As shown in Fig. 1a, the stretch sensor **5** is attached to the subject's torso **2** and is worn at the front side of the subject's body. As shown in Fig. 1b, the transmitting means **6** may be worn at the back side of the subject's body. In use, however, stretch sensor **5** may be worn on other body locations of the subject's torso **2,** such that the system **1** may detect the expansion and contraction of the subject's thorax. Other forms or variations of attaching transmitting means **6** are contemplated, such that the system **1** is processing received signals from the stretch sensor **5.** In another embodiment, stretch sensor **5** and transmitting means **6** may be incorporated into a single, girth like structural unit as illustrated in Fig. 2.

The simplified structure of stretch sensor **5** as shown in Fig. 1a as well as Fig. 1b forms a length of elastic yet resilient ribbon that automatically returns into its original shape after deformation such as stretching or the like. In order to do so, the stretch sensor **5** meets criteria of an elastomer sensor providing at least one, preferably three electrodes thereof. Further, the stretch sensor **5** changes its capacitance when stretched. In another embodiment, stretch sensor **5** may be water-repellent, water-resistant or waterproof to preclude the likelihood of component failure due to water inlet. For the stretch sensor **5** any length between 2 to 25 cm, depending on desired responsiveness desired by the particular application, may be employed from a length sufficient to embrace entirely around the subject's torso **2** to sense for the expansion and contraction of the subject's thorax.

As shown in Fig. 1b in a simplified version, appearance of transmitting means **6** may be miniaturized such that it is not bothering subject in motor activity. In some embodiments, material of transmitting means **6** may be stiff or rigid such that it will not yield to forces contributing to alter its geometry such as composition of single components of transmitting means **6,** illustrated in some embodiment in Fig. 2. Thus, preventing or reducing the likelihood of error or damage thereof. In another embodiment, transmitting means **6** may be water-repellent, water-resistant or waterproof to preclude the likelihood of component failure due to water inlet.

Fig. 1b shows a possible embodiment of a spatially divided receiver unit **7** functioning as alarming device issuing an alert signal. The receiver unit **7** has wireless communication with the transmitting means **6** such that subject is free to move without the requirement of additional elements, such as wires or cable connection. Moreover, the signal is transmitted in real-time from the transmitting means **6** to the receiver unit **7** such that respiratory dysfunction can be detected immediately. In some embodiments, the alert signal issued by the receiver unit **7** may have a characteristic of an audible or visual signal. Other forms or variations of the perceptible pattern as a visual readout may represent a device having a screen, a display a pilot- or warning lamp. Moreover, in some embodiments, spatially receiver unit **7** is a standalone.

Receiver unit **7** is not necessarily attached to the circuit, hence, it may be independently battery powered or may use other energy sources. This simplified structure of receiver unit **7** demonstrates a clear advantage over prior art devices requiring housing circuit or the like. The foregoing greatly simplifies user friendliness and signaling safety on the part of the receiver unit **7** (in case an alert signal is outputted) in terms of using the system **1** for monitoring in natural surroundings without provision or supply of commercial electricity.

With this knowledge, as described in detail above, Fig. 2 shows an enlarged/detailed view of at least minimum requirements of the monitoring device **3** according to embodiments of the present invention. The monitoring device **3** comprises stretch sensor **5** and transmitting means **6.** These are technically connected to each other, for example by a data cable **10.** Transmitting means **6** includes elements of power supply **8, 9** as well as essential components **11-14** of transmitting means **6** such as amplifier **11,** analog-digital converter **12,** evaluation means **13** and emitter **14.** Yet, at some point elements of power supply **8, 9** may not ultimately require a commercial battery. In this way, it can also encompass alternative sources such as solar energy, piezoelectricity, triboelectricity or the like. To some extent, this allows for instance more charging flexibility such that the subject can continue wearing the system **1** while charging or even prevents when intending to wear the system **1** that it is not useable due to flat battery. Components **11-14** communicate with each other or may be integrated into a PCB (printed circuit board) or a microchip and may be coupled to an antenna **15** to transmit the signal. The components **11-14** of the transmitting means **6** may be e.g. integrated in a circuit board or a microchip.

The stretch sensors **5** serves to sense for excursion and contraction of the subject's thorax, wherein a signal is captured preferably at a frequency of 5-50 Hz, more preferably 10-50 Hz. The stretch sensor **5** transmits an analog signal for further processing, wherein the signal is amplified by the amplifier **11** and converted into a digital signal by the analog-digital converter **12.** In that way, the system **1** provides a self-contained structure. In some embodiment, transmitting means **6** can be separated from the stretch sensor **5** providing the user any flexibility such as charging activities.

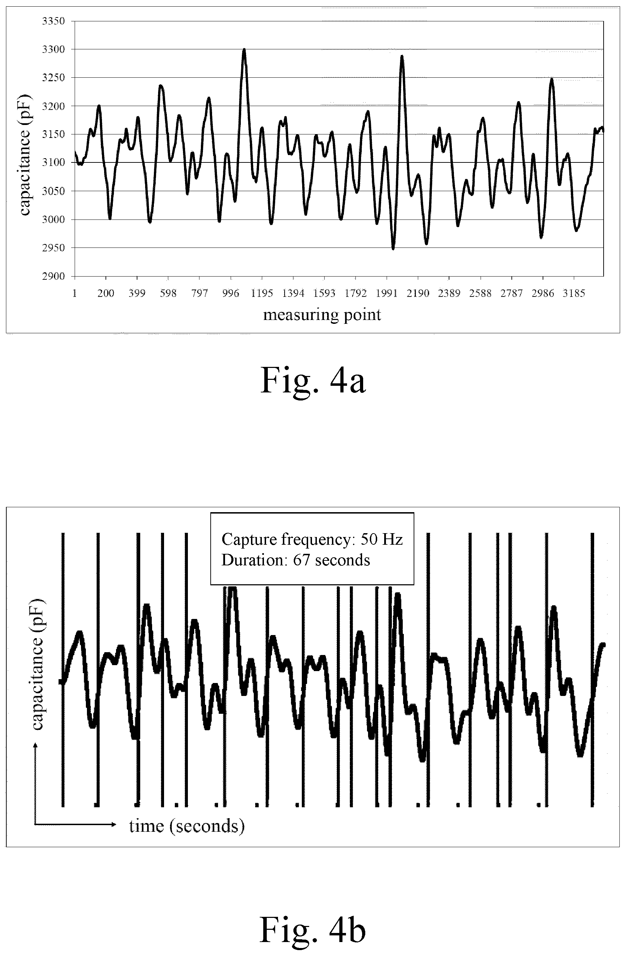

The evaluation means **13** is programmed to evaluate the captured signal from the stretch sensor **5** that changes its capacitance upon stretching. Fig. 4a shows an exemplary illustration of waveforms initially outputted from the stretch sensor **5.** More precisely, the capacitance of the stretch sensor **5** is measured for 67.5 seconds with a capture frequency of 50 Hz. The diagram in Fig. 4a shows the measured capacitance values plotted on the y-axis and the measurement points plotted on the x-axis. The processed and thus digitized signal may be monitored continuously and smoothed by sequentially averaging every capacitance signal with the capacitance values recorded in preceding [N] amount of time, such as second(s). For example, if the capture frequency of the signal is 50 Hz, the last 50 signals are used for averaging in order to take into account all values recorded in the preceding second. This is shown in Fig. 4b, where the average values (y-axis) of the raw capacitance data from Fig. 4a are plotted against the time (x-axis). The time frame of primary smoothing of sequential signals may be chosen such that interference signals not corresponding to thorax excursions are effectively neutralized. In order to neutralize and/or damp short and motion-related excursions, which relate to coughs and the like, mean values of [N] of the sequentially averaged mean capacitance values may be computed.

From this averaged values, the direction which is equivalent to the tangent (ascending, descending) may be calculated from preceding values. This direction indicates the inspiration (ascending) and expiration (descending) and can be used to characterize one breathing cycle, which includes a single inspiration followed by a single expiration. Initial wave parameters are registered, such as troughs, crests, amplitude and the like. As a result, the thoracic inspiration as well as expiration event is assumed to occur and is filed, when the mean value of an ascending or descending signal passes [F]% of the preceding amplitude, reckoned from the turning point (maximum- and minimum value of the capacitance, respectively). The amplitude is computed by subtracting the minimum value of the maximum value of the capacitance during one breathing phase. Note that the amplitudes may change in every breathing cycle, however, they are larger than the intercurrent deflections corresponding to movements, coughs, sneezing, etc. The sequential inspiration frequency is computed continuously and compared to the limit values.

Hence, neutralization of interference signals such as movement of trunk or extremities, coughing, laughing, screaming, sneezing or paradox thoracic excursion is accomplished with the evaluation means **13.** A signal is triggered when a predefined limit value of respiratory rate is underrun. The emitter **14** emits that signal via the antenna **15** to the spatially divided receiver unit **7** in real-time. Any suitable wireless connection may be used, including GSM, a Bluetooth^{®} connection, a wireless local area network connection and the like.

Turning to Fig. 3, monitoring device **3** can be extended by any further components. Monitoring device **3** may include tracking means **16** such that the subject wearing the system **1** can be easily tracked by identifying the geographical location in case an alert signal is issued by the spatially divided receiver unit **7.**

According to the CDC (Centers for Disease Control and Prevention, a National Public Health Institute in the U.S.) every day, about ten people die from unintentional drowning. Of these, two are children aged 14 or younger. Drowning is a leading and though preventable cause of death, in part, due to the absence of validated real-time alert signals extracted from a medical as well as life-sustaining vital function observation. Vital signs, whereby one of them is the respiratory rate, are understood as the primary medical signs. As such, they describe the vital status of the human body and are used to give hints of early warnings signs in emergency situations. Under such circumstances every minute counts, because time is brain. Therefore, it is advisable that the respiratory rate is monitored by a reliable system, which is provided by the object of the present invention.

In essence, breathing is defined as a periodic incorporation of a defined oxygen volume into the body, which is achieved by the respiratory muscles (diaphragm and intercostal muscles). When breathing in, the diaphragm lowers and, simultaneously the ribs rise. This mechanism allows air flowing into the lungs, whose volume increases. Hence, the body volume is proportionally increased, which is particularly measurable at the site of thorax and/or torso. During exhalation, the process is reversed. These periodic changes in body volume, which take place at regular intervals when the body is in a state of rest, can be determined by measuring the excursion of the thorax and/or torso.

In order to do so, the object of the present invention provides a system **1** for monitoring including the measurement of the excursion via stretch sensors **5.** Therefore, a stretch sensor **5,** attached to the subject's torso **2,** possess the capacity to register every excursion of the subject's thorax. The processed signal, which is initially captured by the stretch sensors **5** attached to the subject's torso **2,** draws conclusion about the subject's vital status and, indeed, transmits a real-time alert signal if necessary (in case of emergency) to a spatially divided receiver unit **7.**

By generating the alert signal, the time to find the subject can be shortened considerably. Thus, this increases the chances of survival immensely, particularly without neurological damage. Ideally, less than five minutes should elapse between generating the signal and finding the subject. This cutoff corresponds to the European Resuscitation Council (ERC) Guidelines telling that from about five minutes without oxygen or breathing, a neurodegenerative process begins which is, in most cases irreversible. From that point on, minute by minute, the probability of ROSC (Return of Spontaneous Circulation) decreases drastically and the outcome for the user is ultimately lethal.

Summarizing, the present invention allows to detect and rescue a drowning subject within a few minutes and thus, creates the best conditions to prevent irreversible damages and in the worst case the death of the subject.

### EXAMPLES

Examples were carried out on a healthy adult human volunteer, hereinafter referred to as subject, after informed consent. The subj ect was wearing the system, such that the stretch sensor is attached to the subject's torso in order to possess the capacity to register every excursion and contraction of the subj ect's thorax under given situations or rather simulations. The capacitance of the stretch sensor is measured with a certain capture frequency and the measured capacitance is subsequently transferred to transmitting means for further processing. Therein, evaluation means is configured i) to distinguish between breathing- as well as motor activity or any thereof and ii) to detect pathological breathing patterns causing a triggering event that is further outputted in the form of an alert signal. In order to provide proof-of-concept, both aforementioned configurations are shown in the following two examples in form of raw- and processed data obtained as diagram illustrating actual waveforms.

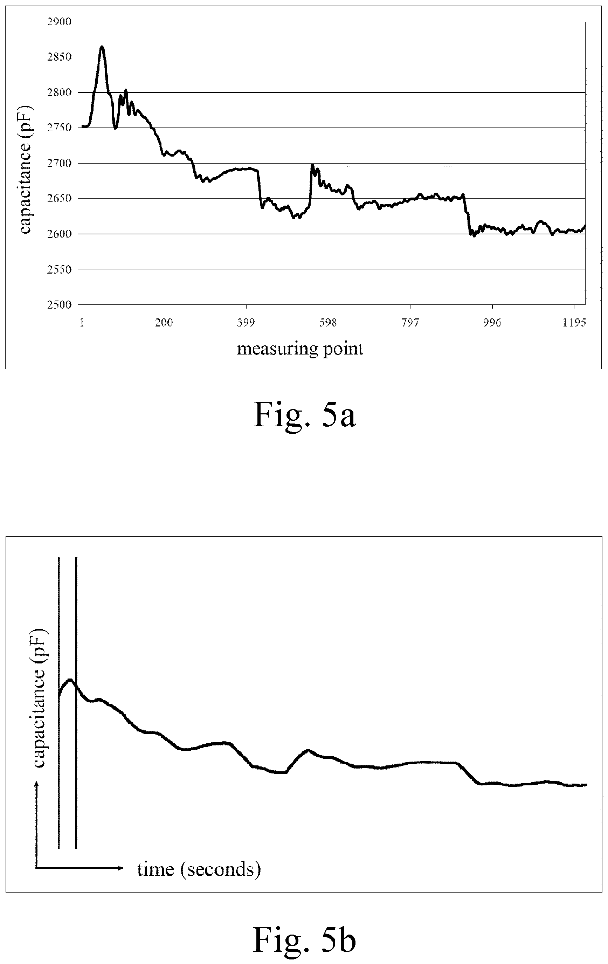

In Fig. 4a and Fig. 5a the diagram's x-axis shows measuring points, whereas the y-axis presents the capacitance in pikoFarad (pF). In Fig. 4b and Fig. 5b the diagram's x-axis shows the time in seconds, whereas the y-axis presents the capacitance in pikoFarad (pF).

### Example 1: Movement and bending at 50 Hz

For this purpose, the subject's respiratory frequency, in particular every inspiration and expiration was detected during motor activity and bending (Fig. 4a and Fig. 4b). The capacitance of the stretch sensor was measured at a capture frequency of 50 Hz for a time span of 67.5 seconds (raw data, Fig. 4a). Thereby, the mean capacitance values of the raw data ([N] value) per second are sequentially determined, meaning that at a capture frequency of 50 Hz the capacitance values 1-50 are averaged followed by the values 2-51, 3-52 and so forth. By taking the mean of the measured capacitance values it is possible to reduce motion-related excursion. While the subject is moving, the stretch sensor is also deformed leading to a change in capacitance which is unrelated to inspiration and expiration. By taking the mean values these artifacts can be smoothed out (Fig. 4b).

Out of these mean values, parameters such as amplitude as well as minimum- and maximum value of the last breathing phase are continuously calculated. This is ascertained by subtracting the minimum- from the maximum value of the last breathing phase thereby achieving the value of one amplitude. In order to determine the occurrence of the next breathing cycle, a predefined percentage, e.g. 40%, of the prior calculated amplitude is used. More precisely, as soon as the amplitude of the current mean values of the breathing phase at hand are above or below the predefined percentage, e.g. 40%, of the amplitude of the last breathing phase, a new breathing cycle will be triggered. Additionally, the last two amplitudes as well as the performance direction (inspiration/expiration) are stored temporarily. Hence, to be illustrated in the diagram (Fig. 4b), vertical lines correspond to one breathing frequency (breathing cycle). Additionally, marked dots at the bottom of the diagram correspond to a five-second interval.

### Example 2: Apnea (no inspiration) at 50 Hz

According to the AASM (American Academy of Sleep Medicine) apnea is defined as the cessation of airflow for at least ten seconds, whereas apnea may last for 30 seconds or even longer. During that phase, muscular activity of the chest wall is either absent or irregular and will not result in any significant excursion of the chest diameter. On the basis of the above described example and in order to simulate a respiratory failure or rather to induce an artificially event of apnea the subject held his breath for 24 seconds. This event became particularly apparent in Fig. 5a and Fig. 5b, whereas Fig. 5a present raw data and Fig. 5b demonstrates smoothened results as described above. To this end, the point in time to trigger a triggering event is reached as soon as the ongoing mean values have exceeded or rather deceeded 40% of the prior calculated amplitude. According to Fig. 5b the last detectable inspiration as well as expiration represents the peak between the two vertical lines. However, after the second vertical line no respiratory activity is measured, hence, a triggering event is induced.

## Claims

1. System (1) for monitoring the thoracic excursion of a subject, the system (1) comprising a monitoring device (2), comprising
• attaching means (4), integratable into a garment,
• a stretch sensor (5), and
• transmitting means (6),
and a receiver unit (7), which is spatially divided from the monitoring device (2),
wherein the stretch sensor (5) is for attachment to the subject's body to detect a signal corresponding to expansion and contraction of the thorax, and
wherein the transmitting means (6) include evaluation means (13) to process the signal obtained from the stretch sensor (5),
wherein the evaluation means (13) is configured to divide the expansion and contraction of the thorax into thorax excursions and interference signals,
wherein the evaluation means (13) is configured to detect a triggering event, wherein the triggering event is reached as soon as the frequency of the thoracic excursion falls out of a predefined frequency range over a predefined time span,
wherein the transmitting means (6) include an emitter (14) to emit a signal to the receiver unit (7) if the triggering event is detected, and
wherein the receiver unit (7) is configured to receive said signal of the emitter (14) and to issue an alert signal.

2. System (1) according to claim 1, wherein the triggering event is a respiratory frequency dysfunction.

3. System (1) according to claim 2, wherein the evaluation means (13) is configured to transmit a signal to the receiver unit (7) if a value of respiratory frequency as an indicator of potential respiratory dysfunction has been detected.

4. System (1) according to any of the claims 1 to 3, wherein the evaluation means (13) is configured to learn about the subject's normally distributed respiratory frequency over a predefined time span.

5. System (1) according to any of the claims 1 to 4, wherein the transmitting means (6) is configured to transmit the signal to the receiver unit (6) wirelessly and in real-time.

6. System (1) according to any of the claims 1 to 5, wherein the alert signal is a perceptible pattern, such as audibly or visually.

7. System (1) according to any of the claims 1 to 6, wherein the receiver unit (7) comprises a visual readout such as a screen, a display, a pilot- or warning lamp.

8. System (1) according to any of the claims 1 to 7, wherein the stretch sensor (5) comprises an elastomer stretch sensor with at least one electrode, preferably three thereof.

9. System (1) according to any of the claims 1 to 8, wherein the garment further comprises a stretchable item of clothing to embrace the torso (2) of the subject's body, such as a belt integrated into a top.

10. System (1) according to any of the claims 1 to 9, wherein the stretch sensor (5) and transmitting means (6) are water-repellent, water-resistant or waterproof.

11. System (1) according to any of the claims 1 to 10, wherein the system (1) further comprises tracking means (16) configured to physically locate current geographical location of the attaching means (4).

12. A method for monitoring the thoracic excursion of a subject, using a system (1) according to any of the claims 1 to 11, comprising the steps:
• perceiving and transmitting expansion and contraction of the subject's thorax;
• dividing the expansion and contraction of the thorax into thorax excursions and interference signals;
• calculating the subject's thoracic excursion;
• determining if the triggering event is induced; and
• transmitting a signal in real-time to the receiver unit (7) which is instantly outputting an alert signal if the triggering event is occurring.

13. The method of claim 12, wherein the evaluation means (13) is subtracting interference signals out of the group comprising muscle activity when moving arms and upper body, laughing, coughing, sneezing, crying or agitation from the expansion and contraction of the thorax.

14. The use of a system (1) of any of the claims 1 to 11 for preventing unintentional fatal drowning.
